(19) **Europäisches Patentamt**

**European Patent Office**

**Office européen des brevets**

(11) **EP 4 144 721 A1**

(12) **EUROPEAN PATENT APPLICATION**
published in accordance with Art. 153(4) EPC

(43) Date of publication:
**08.03.2023 Bulletin 2023/10**

(21) Application number: **21880398.9**

(22) Date of filing: **07.10.2021**

(51) International Patent Classification (IPC):
**C07C 253/30** (2006.01)       **C07C 253/34** (2006.01)
**C07C 255/08** (2006.01)       **B01J 27/228** (2006.01)

(52) Cooperative Patent Classification (CPC):
(C-Sets available)
**C07C 253/30; B01J 27/228; C07C 253/34;**
**Y02P 20/52**                                    (Cont.)

(86) International application number:
**PCT/KR2021/013749**

(87) International publication number:
**WO 2022/080751 (21.04.2022 Gazette 2022/16)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **12.10.2020   KR 20200131025**
           **06.10.2021   KR 20210132515**

(71) Applicant: **Lg Chem, Ltd.**
**Seoul 07336 (KR)**

(72) Inventors:
• **PARK, Si Jung**
  **Daejeon 34122 (KR)**

• **KIM, Wonseok**
  **Daejeon 34122 (KR)**
• **AN, Yujin**
  **Daejeon 34122 (KR)**
• **JUNG, Hyunchul**
  **Daejeon 34122 (KR)**
• **PARK, Sae Hume**
  **Daejeon 34122 (KR)**
• **OH, Wan Kyu**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **PREPARATION METHOD OF ACRYLONITRILE DIMER**

(57)    According to the present disclosure, there is provided a preparation method of an acrylonitrile dimer capable of obtaining an acrylonitrile dimer with high purity and enhancing process efficiency and economic feasibility by easily recovering the catalyst used in the reaction.

EP 4 144 721 A1

**(Cont. next page)**

(52) Cooperative Patent Classification (CPC): (Cont.)

C-Sets
**C07C 253/30, C07C 255/09;**
**C07C 253/34, C07C 255/09**

**Description**

[TECHNICAL FIELD]

Cross-reference to Related Application(s)

This application claims the benefits of Korean Patent Applications No. 10-2020-0131025 filed on October 12, 2020 and No. 10-2021-0132515 filed on October 6, 2021 with the Korean Intellectual Property Office, the disclosure of which is incorporated herein by reference in its entirety.

The present disclosure relates to a preparation method of an acrylonitrile dimer.

[BACKGROUND OF ART]

An acrylonitrile dimer collectively refers to 1,4-dicyano-1-butene (DCB), 1,4-dicyano-2-butene, methylene glutaronitrile (MGN), etc., which are a straight-chain compound having six carbon atoms prepared by dimerization of acrylonitrile. Out of the dimers of acrylonitrile, 1,4-dicyano-1-butene (DCB) and 1,4-dicyano-2-butene are collectively called 1,4-dicyanobutene (DCB). The 1,4-dicyanobutene (DCB) may be converted into adiponitrile through a hydrogenation reaction, which is advantageously used as an intermediate for preparing hexamethylenediamine, a main monomer of nylon 66.

The acrylonitrile dimer may be prepared by reacting acrylonitrile in a solvent capable of donating protons in the presence of a phosphorus-based catalyst. The main product of the dimerization reaction is 1,4-dicyanobutene (DCB), and a small amount of methylene glutaronitrile (MGN) may be prepared as a byproduct. In a reaction mixture after the reaction, components such as MGN, unreacted acrylonitrile, catalyst, solvent, etc., are mixed together with DCB as the main product, and a pure acrylonitrile dimer may be obtained through a purification process.

Since the catalyst used for the preparation of the acrylonitrile dimer is an expensive compound and greatly affects preparation costs, an attempt has been made to recover and reuse the catalyst after the reaction. Accordingly, as a method of purifying the reaction mixture after the acrylonitrile dimerization reaction, a method of separating an acrylonitrile dimer from the catalyst by distillation or extracting the catalyst from the reaction mixture by using an extraction solvent has been proposed.

However, in the case of the distillation method, since a boiling point of the acrylonitrile dimer is as high as about 254 °C, distillation is performed at a high temperature, and thus side reactions may occur. In addition, in the case of the extraction method, the affinity between the acrylonitrile dimer and the catalyst is so high that separation thereof is difficult and a separate extraction solvent is required, and a post-process for recovering the catalyst from the extract after extraction is further required, thereby making an overall preparation process more complicated and causing a problem of rising costs.

Accordingly, there is a need for a method capable of efficiently separating the acrylonitrile dimer and the catalyst during the purification process.

[DETAILED DESCRIPTION OF THE INVENTION]

[Technical Problem]

In the present disclosure, there is provided a preparation method of an acrylonitrile dimer capable of efficiently separating a phosphorus-based catalyst from an acrylonitrile dimer to enhance process efficiency and economic feasibility.

[Technical Solution]

According to one embodiment of the present disclosure, there is provided a preparation method of an acrylonitrile dimer including

a reaction process of preparing an acrylonitrile dimer by reacting a hydrocarbon-based solvent; an alcohol having a boiling point higher than a boiling point of the hydrocarbon-based solvent, capable of phase separation from an acrylonitrile dimer, and having two to six carbon atoms; and acrylonitrile in a presence of a phosphorus-based catalyst,
a first purification process of distilling the reaction mixture to remove unreacted acrylonitrile and the hydrocarbon-based solvent after completion of the reaction process, and
a second purification process of stirring the reaction mixture from which the unreacted acrylonitrile and the hydrocarbon-based solvent are removed, allowing the resulting reaction mixture to stand for phase separation, and sep-

**EP 4 144 721 A1**

arating an alcohol phase including the phosphorus-based catalyst and an acrylonitrile dimer phase.

[ADVANTAGEOUS EFFECTS]

According to the present disclosure, an acrylonitrile dimer can be obtained with high purity, and a catalyst used for a reaction can be easily recovered, thereby enhancing the efficiency and economic feasibility of a preparation process.

[DETAILED DESCRIPTION OF THE EMBODIMENTS]

The terminology used herein is for the purpose of describing particular embodiments only and is not intended to be limiting of the invention. The singular forms are intended to include the plural forms as well, unless the context clearly indicates otherwise. It will be further understood that the terms " include" , " have" , or "possess" when used in this specification, specify the presence of stated features, steps, components, or combinations thereof, but do not preclude the presence or addition of one or more other features, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

Hereinafter, the present invention will be described in detail.

The preparation method of an acrylonitrile dimer according to one embodiment of the present disclosure includes: a reaction process of preparing an acrylonitrile dimer by reacting a hydrocarbon-based solvent; an alcohol having a boiling point higher than a boiling point of the hydrocarbon-based solvent, capable of phase separation from an acrylonitrile dimer, and having two to six carbon atoms; and acrylonitrile in a presence of a phosphorus-based catalyst,

a first purification process of distilling the reaction mixture to remove unreacted acrylonitrile and the hydrocarbon-based solvent after completion of the reaction process, and

a second purification process of stirring the reaction mixture from which the unreacted acrylonitrile and the hydrocarbon-based solvent are removed, allowing the resulting reaction mixture to stand for phase separation, and separating an alcohol phase including the phosphorus-based catalyst and an acrylonitrile dimer phase.

In a conventional preparation method of an acrylonitrile dimer, a method of distilling the acrylonitrile dimer per se or extracting the catalyst from the reaction mixture by using a separate extraction solvent has been used to separate the acrylonitrile dimer and the catalyst after the dimerization reaction. However, the above method has problems such as complicated process, requiring a lot of manufacturing equipment, high costs, poor purification efficiency, etc.

Accordingly, in the preparation method of an acrylonitrile dimer of the present disclosure, an alcohol having a boiling point higher than that of the hydrocarbon-based solvent and capable of phase separation from the acrylonitrile dimer is used as a co-solvent together with the hydrocarbon-based solvent, thereby enhancing the efficiency and economic feasibility of the purification process.

In other words, the alcohol used in the present disclosure serves as a proton-donating solvent during the dimerization reaction of acrylonitrile, and has a boiling point higher than that of the hydrocarbon-based solvent. Therefore, it is not volatilized together in a first purification process of removing the unreacted acrylonitrile and the hydrocarbon-based solvent after the dimerization reaction.

In addition, since the alcohol causes the phase separation from the acrylonitrile dimer, the alcohol may serve as an extraction solvent for extracting the catalyst in a second purification process. As such, the alcohol is both a reaction solvent and an extraction solvent at the same time, and thus the catalyst included in an alcohol phase after extraction may be recycled to a reaction step together with the alcohol without a separate purification process.

Thus, according to the present disclosure, side reactions caused by distillation at a high temperature may be prevented, the use of a separate extraction solvent and a removal process thereof are unnecessary, and the acrylonitrile dimer and the catalyst may be separated by a simplified process compared to the conventional method, thereby remarkably enhancing the efficiency, convenience and economic feasibility of the process.

As the alcohol, an aliphatic, alicyclic, or aromatic alcohol having two to six carbon atoms may be used. As one example, the alcohol used herein may be cyclohexanol, ethylene glycol, or a combination thereof, preferably cyclohexanol or ethylene glycol, and more preferably cyclohexanol.

In addition, the alcohol may have a boiling point higher than that of the hydrocarbon-based solvent by at least 40 °C, and preferably at least 50 °C or at least 60 °C.

When there is a little difference in boiling points between the alcohol and the hydrocarbon-based solvent, the alcohol may be distilled together in the first purification process without any remaining alcohol to be used as an extraction solvent in the second refining process, and thus it is preferable that the boiling points differ by 50 °C or more. The upper

4

limit of the difference in boiling points between the alcohol and the hydrocarbon-based solvent may not be particularly limited, but may be, for example, 180 °C or less, 110 °C or less, or 90 °C or less.

In addition, the alcohol may preferably have a density of 0.965 g/cm$^3$ or less, or 0.962 g/cm$^3$ or less, and 1.05 g/cm$^3$ or more, or 1.10 g/cm$^3$ or more. Most of the dimers produced by the dimerization reaction of acrylonitrile may be 1,4-dicyanobutene having a density of about 1.0 g/cm$^3$, and thus the phase separation from the acrylonitrile dimer may smoothly occur when the alcohol satisfies the above range of density.

Meanwhile, the alcohol may need to be capable of extracting the phosphorus-based catalyst from the acrylonitrile dimer, and thus the affinity for the phosphorus-based catalyst may need to be higher than that of the acrylonitrile dimer. Accordingly, an alcohol-adiponitrile partition coefficient ($K_{ROH/ADN}$) of the phosphorus-based catalyst represented by the following Equation 1 at 50 °C under a normal pressure (760 torr) may be 1.0 or more, and preferably 1.07 or more, 1.35 or more, or 1.5 or more. The upper limit of the partition coefficient may not be particularly limited, but may be, for example, 6.0 or less, 5.0 or less, or 4.8 or less.

$$[Equation\ 1]$$

$$K_{ROH/ADN} = C_{ROH}/C_{ADN}$$

in above Equation 1,

$C_{ROH}$ is a percent concentration of the phosphorus-based catalyst in alcohol, and
$C_{ADN}$ is a percent concentration of the phosphorus-based catalyst in adiponitrile.

The hydrocarbon-based solvent may be used as a main solvent for the acrylonitrile dimerization reaction. As the hydrocarbon-based solvent, aromatic hydrocarbons, aliphatic hydrocarbons, alicyclic hydrocarbons, etc., having 5 to 15 carbon atoms may be used.

It is preferable that the hydrocarbon-based solvent may have a boiling point of 115 °C or less, or 110 °C or less and 20 °C or more, 30 °C or more, or 50 °C or more, so as to be smoothly removed by distillation together with unreacted acrylonitrile monomers (boiling point of 77 °C) after the completion of the acrylonitrile dimerization reaction. The hydrocarbon-based solvent may be at least one selected from the group consisting of benzene, toluene, n-pentane, n-hexane, cyclopentane and cyclohexane.

According to one preferred embodiment, toluene may be used as the hydrocarbon-based solvent, and cyclohexanol or ethylene glycol may be used as the alcohol. Toluene has a boiling point of about 110 °C and may be volatilized by distillation under relatively mild conditions. Cyclohexanol and ethylene glycol have a boiling point higher than that of toluene by 50 °C or more, and thus may not be volatilized in the first purification process. In addition, cyclohexanol and ethylene glycol may have a phase separation from the acrylonitrile dimer, and have a high solubility to the phosphorus-based catalyst. Thus, they may be suitably used as an extraction solvent for separating the acrylonitrile dimer and the catalyst in the second purification process.

When the proton donating solvent is insufficient during the acrylonitrile dimerization reaction, the polymerization reaction of acrylonitrile may be accelerated. Accordingly, the alcohol may be used 3% by volume or more, 5% by volume or more, or 10% by volume or more, and 50% by volume or less, 40% by volume or less, or 30% by volume or less based on 100% by volume of the hydrocarbon-based solvent in the reaction process. When the content range is satisfied, the acrylonitrile dimerization reaction and the extraction process after the reaction may be smoothly performed.

The phosphorus-based catalyst used in the acrylonitrile dimerization reaction of the present disclosure may be an organophosphorus (III) compound containing at least one hydrocarbyl; and at least one alkoxy or cycloalkoxy.

As one example, the phosphorus-based catalyst may be a phosphonite-based compound represented by (R)$_2$P(OR') or a phosphinite-based compound represented by (R)P(OR')$_2$. In the above formulas (R)$_2$P(OR') and (R)P(OR')$_2$, R and R' may each independently be C1-20 alkyl, C6-20 aryl, C7-20 alkylaryl, C7-20 aralkyl, or C3-20 alkyl. Preferably, R may each independently be C1-20 alkyl or C6-20 aryl, and R' may be each independently be C1-20 alkyl.

Preferably, the phosphorus-based catalyst used herein may be at least one selected from the group consisting of ethyl diphenylphosphinite, isopropyl diphenylphosphinite, ethyl phenylethyl phosphinite, and isopropyl ditolyl phosphinite.

In the reaction process, the phosphorus-based catalyst may be included in a content of 2 mol% or more, 3 mol% or more, or 4 mol% or more, and 8 mol% or less, 7 mol% or less, or 6 mol% or less based on 100 mol% of the acrylonitrile monomer. When the phosphorus-based catalyst is used within the above molar content with respect to the acrylonitrile monomer, side reactions may be suppressed while the acrylonitrile dimerization reaction smoothly proceeds.

In the preparation method of an acrylonitrile dimer of the present disclosure, the reaction process for dimerizing acrylonitrile may be performed at 0 °C to 70 °C under a normal pressure (700 to 800 torr), and preferably at 10 °C to 50 °C, or 20 °C to 40 °C. When a reaction temperature is less than 0 °C, a reaction rate may be low and thus productivity may be reduced, and when the reaction temperature exceeds 70 °C, the reaction rate may become excessively high, and thus the acrylonitrile monomer and dimer may be polymerized into a polymer.

When the reaction process is completed, a first purification process of distilling the reaction mixture to remove the unreacted acrylonitrile and the hydrocarbon-based solvent, having a relatively low boiling point, is performed. In this case, the distilled unreacted acrylonitrile and hydrocarbon-based solvent may be recovered and reused in the reactor.

The conditions of the first purification process may be adjusted according to the boiling point of the hydrocarbon-based solvent used. In one embodiment, the first purification process may be performed under a pressure of 760 torr or less, 400 torr or less, 100 torr or less, or 10 torr or less. The temperature of the first purification process may be appropriately adjusted according to pressure conditions, and may be, for example, in the range of 100 °C or less, 70 °C or less, or 60 °C or less, and 10 °C or more, or 30 °C or more. Under such conditions, only the unreacted acrylonitrile and hydrocarbon-based solvent may be selectively removed without evaporation of the alcohol.

Then, a second purification process of stirring the reaction mixture from which the unreacted acrylonitrile and the hydrocarbon-based solvent are removed, and then subjecting the resulting mixture to phase separation to separate an alcohol phase and an acrylonitrile dimer phase is performed.

Since the alcohol has a boiling point higher than that of the hydrocarbon-based solvent and thus most thereof is not volatilized in the first purification process, the phosphorus-based catalyst may be extracted by using alcohol present in the reaction mixture without further adding alcohol.

The second purification process may be performed at a temperature of 10 to 30 °C, or 20 to 25 °C. When the temperature of the second purification process is too high, the separation of the alcohol phase and the acrylonitrile dimer phase may not occur well, and the ratio of the phosphorus-based catalyst dissolved in the acrylonitrile dimer phase may increase to reduce the separation efficiency. Thus, it is preferable that the second purification process may be performed at room temperature.

Accordingly, after sufficiently mixing the reaction mixture within the above temperature range, the mixture is allowed to stand for phase separation, after which a supernatant and a lower layer solution are separated to complete the extraction.

In addition, in order to further enhance the recovery rate of the catalyst, the process of extraction by further adding the alcohol to the acrylonitrile dimer separated by the second purification process may be repeated.

Most of the acrylonitrile dimer phase separated from the alcohol phase through the above process may consist of 1,4-dicyanobutene (DCB) and may include a small amount of methylene glutaronitrile (MGN). Accordingly, in order to remove the MGN, the acrylonitrile dimer phase is further purified and a partial hydrogenation reaction is performed to prepare adiponitrile, a precursor of hexamethylenediamine.

Meanwhile, the alcohol phase containing the phosphorus-based catalyst may be recovered and reused in a reaction vessel. In the present disclosure, the alcohol used as a co-solvent in the reaction step is used in the second purification process for extracting the phosphorus-based catalyst without using a separate extraction solvent, and thus the process of removing the extraction solvent for reuse of the catalyst may be unnecessary. In addition, not only the catalyst but also the alcohol as the co-solvent may be reused together, thereby reducing preparation costs and increasing process efficiency.

Hereinafter, preferred examples will be suggested for better understanding of the present invention, but the following examples are provided only for the purpose of illustrating the present invention, and it is apparent to those having ordinary skill in the art that various changes and modifications are possible within the scope and technical ideas of the present invention and such changes and modifications are within the appended claims.

**[Example]**

**<Evaluation of partition coefficient of catalyst>**

In order to confirm a partition coefficient of a phosphorus-based catalyst with respect to alcohol and acrylonitrile dimer, a following experiment was performed assuming that unreacted acrylonitrile and hydrocarbon-based solvent were removed from a reaction mixture through a first purification process.

(1) 0.634 g (25 wt%) of ethyl diphenylphosphinite ($Ph_2POEt$) and 2 ml (1.902 g, 75 wt%) of adiponitrile (ADN) were mixed and used as a simulated solution of the reaction mixture from which the unreacted acrylonitrile and the hydrocarbon-based solvent were removed after the first purification step.
(2) The 2 ml of the alcohol shown in table 1 below was added to the simulated solution, and stirred while being heated to 50 °C.
(3) After stirring at 50 °C for five minutes, stirring was stopped and allowed to stand.
(4) After phase separation, 0.25 ml of the supernatant and the lower layer solution was obtained, respectively, and components were analyzed by gas chromatography (GC, SIMADAZU, GC-2030). In this case, a detector was FID and set to 350 ° C, and HP-5MS was used for a column and set to 40-280 ° C. A temperature of an injection port was 260 ° C and $N_2$ was used as a mobile phase.

(5) The percent concentration of the catalyst included in each layer (ADN layer and alcohol layer) and the partition coefficient calculated therefrom are shown in table 1 below.

[Table 1]

| Alcohol | Catalyst concentration in alcohol layer ($C_{ROH}$) | Catalyst concentration in ADN layer ($C_{ADN}$) | Partition coefficient ($C_{ROH}/C_{ADN}$) |
|---|---|---|---|
| Cyclohexanol | 0.1628 | 0.1037 | 1.57 |
| Ethylene glycol | 0.1486 | 0.1077 | 1.38 |
| 1-octanol | 0.1303 | 0.1616 | 0.81 |
| 1-nonanol | 0.0778 | 0.1693 | 0.46 |
| Isopropanol | Layer separation from ADN does not occur well. | | |

**<Preparation of acrylonitrile dimer>**

**Example 1**

An acrylonitrile dimerization reaction was performed as follows by using toluene as a solvent, cyclohexanol (boiling point of 161.8 °C and density of 0.962 g/cm$^3$) as an alcohol co-solvent, and ethyl diphenylphosphinite (Ph$_2$POEt) as a catalyst.

Toluene, acrylonitrile, and cyclohexanol were added to a 1 L reactor at a volume ratio of 10:3:3, and ethyl diphenylphosphinite was added in an amount of 5 mol% based on the acrylonitrile. The mixture was reacted while being stirred at room temperature (25 °C) for 24 hours.

After completion of the reaction, the reaction mixture was distilled at a temperature of 60 °C and under a pressure of 10 torr to remove toluene and unreacted acrylonitrile from the reaction mixture. After that, the reaction mixture was stirred to mix, and then the stirring was stopped and allowed to stand for phase separation. After the phase separation was completed, the supernatant and the lower layer solution were separated, and the components of the supernatant and the lower layer solution were analyzed by using gas chromatography (GC, SIMADAZU, GC-2030). The detector was FID and set to 350 ° C, and HP-5MS was used for a column and set to 40-280 ° C. A temperature of an injection port was 260 ° C, $N_2$ was used as a mobile phase, and 1 ml of the sample was injected.

As a result of the analysis, a detection amount was calculated compared to a sample input amount. It was confirmed that the lower layer solution includes 78.9 wt% of 1,4-dicyanobutene (DCB), 2.1 wt% of methylene glutaronitrile (MGN), 11.9 wt% of cyclohexanol, and 7.1 wt% of ethyl diphenylphosphinite, and it was also confirmed that the supernatant includes 81.3% of cyclohexanol, 10.3 wt% of ethyl diphenylphosphinite, 8.2 wt% of 1,4-dicyanobutene (DCB), and 0.2 wt% of methylene glutaronitrile (MGN).

**Example 2**

An acrylonitrile dimerization reaction was performed as follows by using toluene as a solvent, ethylene glycol (boiling point of 197 °C and density of 1.110 g/cm$^3$) as an alcohol co-solvent, and ethyl diphenylphosphinite (Ph$_2$POEt) as a catalyst.

Toluene, acrylonitrile, and ethylene glycol were added to a 1 L reactor at a volume ratio of 10:3:3, and ethyl diphenylphosphinite was added in an amount of 5 mol% based on the acrylonitrile. The mixture was reacted while being stirred at room temperature (25 °C) for 24 hours.

Thereafter, the first and second purification processes were performed in the same manner as in Example 1, and GC analysis was performed.

As a result of the analysis, a detection amount was calculated compared to a sample input amount. It was confirmed that the lower layer solution includes 76.9 wt% of ethylene glycol, 9.1 wt% of ethyl diphenylphosphinite, 13.6 wt% of 1,4-dicyanobutene (DCB), and 0.4 wt% of methylene glutaronitrile (MGN), and the supernatant includes 67.2 wt% of 1,4-dicyanobutene (DCB), 1.8 wt% of methylene glutaronitrile (MGN), 23.7 wt% of ethylene glycol, and 7.3 wt% of ethyl diphenylphosphinite.

**Comparative Example 1**

The acrylonitrile dimerization reaction was performed as follows by using toluene as a solvent, isopropyl alcohol (boiling point of 82.5 °C and density of 0.786 g/cm$^3$) and formamide (boiling point of 210 °C and density of 1.130 g/cm$^3$) as a co-solvent, and ethyl diphenylphosphinite as a catalyst.

Toluene, acrylonitrile, isopropyl alcohol and formamide were added to a 1 L reactor at a volume ratio of 10:3:1:1, and ethyl diphenylphosphinite was added in an amount of 5 mol% based on the acrylonitrile. The mixture was reacted while being stirred at room temperature (25 °C) for 24 hours.

After completion of the reaction, the reaction mixture was distilled at a temperature of 60 °C and under a pressure of 10 torr to remove toluene, isopropyl alcohol and unreacted acrylonitrile from the reaction mixture. After that, the reaction mixture was stirred to mix, and then the stirring was stopped to stand, but phase separation of formamide and DCB did not occur.

**Comparative Example 2**

An acrylonitrile dimerization reaction was performed as follows by using toluene as a solvent, octanol (boiling point of 185 °C and density of 0.824 g/cm$^3$) as an alcohol co-solvent, and ethyl diphenylphosphinite (Ph$_2$POEt) as a catalyst.

Toluene, acrylonitrile, and octanol were added to a 1 L reactor at a volume ratio of 10:3:3, and ethyl diphenylphosphinite was added in an amount of 5 mol% based on the acrylonitrile. The mixture was reacted while being stirred at room temperature (25 °C) for 24 hours.

Thereafter, the first and second purification processes were performed in the same manner as in Example 1, and GC analysis was performed.

As a result of the analysis, a detection amount was calculated compared to a sample input amount. It was confirmed that the lower layer solution includes 11.1 wt% of octanol, 10.5 wt% of ethyl diphenylphosphinite, 75.8 wt% of 1,4-dicyanobutene (DCB), and 2.6 wt% of methylene glutaronitrile (MGN), and the supernatant includes 9.2 wt% of 1,4-dicyanobutene (DCB), 0.3 wt% of methylene glutaronitrile (MGN), 81.9 wt% of octanol, and 8.6 wt% of ethyl diphenylphosphinite.

**Comparative Example 3**

An acrylonitrile dimerization reaction was performed as follows by using toluene as a solvent, nonanol (boiling point of 214 °C and density of 0.827 g/cm$^3$) as an alcohol co-solvent, and ethyl diphenylphosphinite (Ph$_2$POEt) as a catalyst.

Toluene, acrylonitrile, and nonanol were added to a 1 L reactor at a volume ratio of 10:3:3, and ethyl diphenylphosphinite was added in an amount of 5 mol% based on the acrylonitrile. The mixture was reacted while being stirred at room temperature (25 °C) for 24 hours.

Thereafter, the first and second purification processes were performed in the same manner as in Example 1, and GC analysis was performed.

As a result of the analysis, a detection amount was calculated compared to a sample input amount. It was confirmed that the supernatant includes 85.4 wt% of nonanol, 5.8 wt% of ethyl diphenylphosphinite, 8.5 wt% of 1,4-dicyanobutene (DCB), and 0.3 wt% of methylene glutaronitrile (MGN), and the lower layer solution includes 74.6 wt% of 1,4-dicyanobutene (DCB), 2.2 wt% of methylene glutaronitrile (MGN), 10.3 wt% of nonanol, and 12.9 wt% of ethyl diphenylphosphinite.

**Claims**

1. A preparation method of an acrylonitrile dimer, the method comprising:

    a reaction process of preparing an acrylonitrile dimer by reacting a hydrocarbon-based solvent; an alcohol having a boiling point higher than a boiling point of the hydrocarbon-based solvent, capable of phase separation from an acrylonitrile dimer, and having two to six carbon atoms; and acrylonitrile in a presence of a phosphorus-based catalyst,
    a first purification process of distilling the reaction mixture to remove unreacted acrylonitrile and the hydrocarbon-based solvent after completion of the reaction process, and
    a second purification process of stirring the reaction mixture from which the unreacted acrylonitrile and the hydrocarbon-based solvent are removed, allowing the resulting reaction mixture to stand for phase separation, and separating an alcohol phase comprising the phosphorus-based catalyst and an acrylonitrile dimer phase.

2. The preparation method of an acrylonitrile dimer of claim 1,
   wherein a boiling point of the alcohol is higher than a boiling point of the hydrocarbon-based solvent by 40 °C or more.

3. The preparation method of an acrylonitrile dimer of claim 1,
   wherein the alcohol is cyclohexanol, ethylene glycol, or a combination thereof.

4. The preparation method of an acrylonitrile dimer of claim 1,
   wherein the hydrocarbon-based solvent has a boiling point of 115 °C or less.

5. The preparation method of an acrylonitrile dimer of claim 1,
   wherein the hydrocarbon-based solvent is at least one selected from the group consisting of benzene, toluene, n-pentane, n-hexane, cyclopentane and cyclohexane.

6. The preparation method of an acrylonitrile dimer of claim 1,
   wherein the hydrocarbon-based solvent is toluene and the alcohol is cyclohexanol.

7. The preparation method of an acrylonitrile dimer of claim 1,
   wherein the alcohol is used in an amount of 3% by volume to 50% by volume based on 100% by volume of the hydrocarbon-based solvent in the reaction process.

8. The preparation method of an acrylonitrile dimer of claim 1,
   wherein the phosphorus-based catalyst is at least one selected from the group consisting of ethyl diphenylphosphinite, isopropyl diphenylphosphinite, ethyl phenylethyl phosphinite, and isopropyl ditolyl phosphinite.

9. The preparation method of an acrylonitrile dimer of claim 1,
   wherein the unreacted acrylonitrile and the hydrocarbon-based solvent separated in the first purification process; and the alcohol phase comprising the phosphorus-based catalyst separated in the second purification process are recycled to the reaction process.

<table>
<tr><td colspan="2">**INTERNATIONAL SEARCH REPORT**</td><td>International application No.<br>**PCT/KR2021/013749**</td></tr>
</table>

**A. CLASSIFICATION OF SUBJECT MATTER**

**C07C 253/30**(2006.01)i; **C07C 253/34**(2006.01)i; **C07C 255/08**(2006.01)i; **B01J 27/228**(2006.01)i

According to International Patent Classification (IPC) or to both national classification and IPC

**B. FIELDS SEARCHED**

Minimum documentation searched (classification system followed by classification symbols)

C07C 253/30(2006.01); B01J 31/18(2006.01); B01J 31/30(2006.01); B01J 38/70(2006.01); C07C 255/08(2006.01); C07C 41/54(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아크릴로니트릴(acrylonitrile), 이량체(dimer), 용매(solvent), 알코올(alcohol), 촉매(catalyst)

**C. DOCUMENTS CONSIDERED TO BE RELEVANT**

| Category* | Citation of document, with indication, where appropriate, of the relevant passages | Relevant to claim No. |
|---|---|---|
| Y | US 3732281 A1 (FELDMAN, J. et al.) 08 May 1973 (1973-05-08)<br>See claim 1; columns 2-4; and example 3. | 1-9 |
| Y | JP 2003-081901 A (MITSUBISHI CHEMICALS CORP.) 19 March 2003 (2003-03-19)<br>See claims 1 and 3; and paragraphs [0005] and [0031]. | 1-9 |
| A | EP 0010886 A1 (IMPERIAL CHEMICAL INDUSTRIES LIMITED) 14 May 1980 (1980-05-14)<br>See claims 1-10. | 1-9 |
| A | US 4952541 A (HECKLE, W. A. et al.) 28 August 1990 (1990-08-28)<br>See entire document. | 1-9 |
| A | KR 10-1993-0019611 A (UBE IND. LTD) 18 October 1993 (1993-10-18)<br>See entire document. | 1-9 |

| ☐ Further documents are listed in the continuation of Box C. | ☑ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2022** | **25 January 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2021/013749**

| Patent document cited in search report | | | Publication date (day/month/year) | Patent family member(s) | | | Publication date (day/month/year) |
|---|---|---|---|---|---|---|---|
| US | 3732281 | A1 | 08 May 1973 | None | | | |
| JP | 2003-081901 | A | 19 March 2003 | JP | 4019670 | B2 | 12 December 2007 |
| EP | 0010886 | A1 | 14 May 1980 | EP | 0010886 | B1 | 01 December 1982 |
| | | | | JP | 55-098149 | A | 25 July 1980 |
| | | | | JP | 63-064418 | B | 12 December 1988 |
| | | | | US | 04316857 | A | 23 February 1982 |
| US | 4952541 | A | 28 August 1990 | None | | | |
| KR | 10-1993-0019611 | A | 18 October 1993 | CN | 1033700 | C | 01 January 1997 |
| | | | | CN | 1079463 | A | 15 December 1993 |
| | | | | EP | 0559168 | A2 | 08 September 1993 |
| | | | | EP | 0559168 | B1 | 29 May 1996 |
| | | | | JP | 06-092923 | A | 05 April 1994 |
| | | | | JP | 06-145130 | A | 24 May 1994 |
| | | | | JP | 06-157445 | A | 03 June 1994 |
| | | | | JP | 2724650 | B2 | 09 March 1998 |
| | | | | JP | 2830965 | B2 | 02 December 1998 |
| | | | | JP | 3033872 | B2 | 17 April 2000 |
| | | | | KR | 10-1995-0014226 | B1 | 23 November 1995 |
| | | | | US | 5332844 | A | 26 July 1994 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200131025

- KR 1020210132515